# EUROPEAN PATENT APPLICATION

(11) **EP 4 491 706 A1**
(43) Date of publication of application: **15.01.2025**
(21) Application number: 23766820.7
(22) Date of filing: 07.03.2023
(51) Int. Cl.: C12N 1/14, B01D 37/02, B01D 61/14, B01J 20/14, C12N 9/42

(54) **FILTER AID, FILTERING PROCESSING METHOD, AND CELLULASE PRODUCTION METHOD**

(30) Priority: 08.03.2022 JP 2022034963
(71) Applicant: Toray Industries, Inc., Tokyo 103-8666 (JP)
(72) Inventor: NOGUCHI, Takuya, Kamakura-shi, Kanagawa 248-8555 (JP); KAGAWA, Yusuke, Kamakura-shi, Kanagawa 248-8555 (JP); YAMADA, Katsushige, Kamakura-shi, Kanagawa 248-8555 (JP)
(74) Representative: Kador & Partner Part mbB
(86) International application number: PCT/JP2023/008470
(87) International publication number: WO 2023/171644

(57) **Abstract**

In the filtration treatment for removing fungal cells and suspended matters from a cellulase-containing culture liquid in a method for manufacturing cellulase, a mixture of an inorganic filter aid and a culture liquid of filamentous fungus is used as a filter aid to obtain cellulase with its enzymatic activity maintained at a high level.

## Description

### Technical Field

The present invention relates to a filter aid containing a mixture of an inorganic filter aid and a culture liquid of a filamentous fungus as an active ingredient, a method of filtration treatment using the filter aid, and a method of manufacturing cellulase using the filter aid.

### Background Art

A filamentous fungus of the genus *Trichoderma* is known to have a high protein production capacity, and a method of producing protein using the filamentous fungus of the genus *Trichoderma* has been investigated. The filamentous fungus of the genus *Trichoderma* utilizes cellulose, lactose, cellobiose, or the like as an inducer to produce cellulase, which is classified as a saccharifying enzyme among proteins.

Cellulase is used as a saccharifying enzyme to hydrolyze a cellulose-containing biomass to produce a sugar. As a first step for obtaining cellulase from a cellulase-containing culture liquid of the filamentous fungus of the genus *Trichoderma,* cells of the filamentous fungus of the genus *Trichoderma* and suspended matters in the culture liquid are removed from the cellulase-containing culture liquid of filamentous fungus of the genus *Trichoderma.* By removing them, membrane clogging in the downstream membrane filtration steps (MF, UF) can be avoided. There is a method of filtering with a suction filtration device such as a precoat filter or a pressure filtration device such as a filter press, using a filter aid, as a means of removing suspended matters, and a method using diatomaceous earth or perlite, especially diatomaceous earth as a filter aid is known (Non-Patent Literature 1). In such cases, a filter aid including biodegradable fibers such as cellulose or chitin as main ingredients may be used (Patent Documents 1 and 2).

There are two methods to use the filter aid: a body-feed method in which a filter aid is directly added to a liquid to be filtered, and a precoat method in which a layer of filter aid is previously formed on a surface of a filter medium. Filtration using both methods is usually used in solid-liquid separation using a filter aid (Patent Literature 3).

### Citation List

### Patent Literature

Patent Literature 1 WO 2013/018678
Patent Literature 2 JP-H8-224077A
Patent Literature 3 JP-2019-176837A

### Non-Patent Literature

Tomantschger Kurt et al., MATHEMATICAL MODEL FOR THE PARTICLE SIZE DISTRIBUTION OF A KIESELGUHR FILTER GRANULATION, METALURGIA INTERNATIONAL, 17, 10, 192 - 197, 2012

### Summary of Invention

### Technical Problem

In the process of investigating filtration treatment as a solid-liquid separation method to remove microorganism cells and suspended matters from the cellulase-containing culture liquid, the inventor has found that the enzymatic activity of cellulase recovered after filtration treatment was significantly lower than that before the filtration treatment. Decreased enzymatic activity of cellulase leads to decreased degradation potential of a cellulose-containing biomass and causes a decrease in sugar yield when the cellulose-containing biomass is hydrolyzed with cellulase to produce a sugar.

Therefore, an object of the present invention is to establish a method of maintaining enzymatic activity at a high level after filtration treatment of a cellulase-containing culture liquid of microorganism.

### Solution to Problem

As a result of intensive study to solve the above-described problem, the present inventor has newly found that a mixture of an inorganic filter aid such as diatomaceous earth and a culture liquid of filamentous fungus such as the filamentous fungus of the genus *Trichoderma* or the genus *Talaromyces* is useful as a filter aid, and cellulase with its enzymatic activity maintained at a high level can be recovered by performing filtration treatment using the filter aid as the above-mentioned solid-liquid separation step, and has attained the present invention.

That is, the present invention is constituted by the following [1] to [16].
[1] A filter aid which is a mixture of an inorganic filter aid and a culture liquid of a filamentous fungus.
[2] The filter aid according to [1], wherein the inorganic filter aid is diatomaceous earth.
[3] The filter aid according to [1] or [2], wherein the filamentous fungus is the genus *Trichoderma* or the genus *Talaromyces.*
[4] The filter aid according to any one of [1] to [3], wherein the culture liquid is a culture liquid cultured until a dry cell weight (g-cell/L) of the filamentous fungal cells reaches at least 1.0 g/L or more.
[5] The filter aid according to any one of [1] to [4], wherein the culture liquid contains filamentous fungal cells after culturing.
[6] A method of filtration treatment, including a step of filtration treatment together with the filter aid according to any one of [1] to [5].
[7] The method of filtration treatment according to claim 6, wherein the filtration treatment step is a step in which the filter aid according to any one of [1] to [5] is added to a liquid to be filtered.
[8] The method of filtration treatment according to [6] or [7], wherein the filtration treatment step is a filtration treatment step in which the liquid to be filtered is passed through a filter medium precoated with the filter aid according to any one of [1] to [5] from a precoated side thereof.
[9] A method of manufacturing cellulase, including:
   a step (1) of culturing a microorganism capable of producing cellulase; and
   a step (2) of performing filtration treatment of a culture liquid containing microorganism cells after culturing obtained in the step (1) together with the filter aid according to any one of [1] to [5], prepared independently of the step (1), to recover cellulase from which the microorganism cells have been filtered off.
[10] The method of manufacturing cellulase according to [9], wherein the microorganism in the step (1) is a microorganism capable of producing cellulase having β-xylosidase activity, and the step (2) is a step of recovering cellulase having β-xylosidase activity.
[11] The method of manufacturing cellulase according to [9] or [10], wherein the filtration treatment in the step (2) is a filtration treatment in which the filter aid according to any one of claims 1 to 5, prepared independently of the step (1), is added to the culture liquid containing microorganism cells after culturing obtained in the step (1).
[12] The method of manufacturing cellulase according to any one of [9] to [11], wherein the filtration treatment in the step (2) is a filtration treatment in which the culture liquid containing microorganism cells after culturing obtained in the step (1) is passed through a filter medium precoated with the filter aid according to any one of [1] to [5], prepared independently of the step (1), from a precoated side thereof.
[13] The method of manufacturing cellulase according to any one of [9] to [12], wherein the filtration treatment in the step (2) is a filter press treatment.
[14] The method of manufacturing cellulase according to any one of [9] to [13], wherein the microorganism capable of producing cellulase, which is to be cultured in the step (1), is a filamentous fungus of the genus *Trichoderma* or the genus *Talaromyces.*
[15] The method of manufacturing cellulase according to [14], including a step of mixing a preculture liquid of the filamentous fungus to be cultured in the step (1) and the inorganic filter aid to prepare the filter aid according to any one of [1] to [5].
[16] A method of manufacturing a sugar, including:
   a step of manufacturing cellulase by the method according to any one of [9] to [15]; and
   a step of hydrolyzing a cellulose-containing biomass with the cellulase obtained in the preceding step.

### Advantageous Effects of Invention

According to the present invention, cellulase with its enzymatic activity maintained at a high level can be recovered by filtration treatment of a cellulase-containing culture liquid.

### Brief Description of Drawings

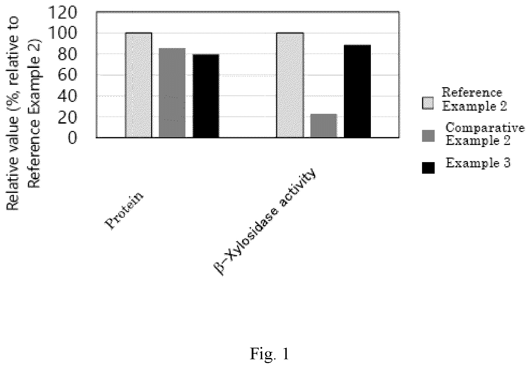

FIG. 1 shows the relative values of the protein concentration (g/L) and specific activity of β-xylosidase (U/mg-protein) of the filtrates recovered by the filtration treatment in Comparative Example 2 and Example 3, where the protein concentration and β-xylosidase activity of an unfiltered crude enzyme liquid derived from the filamentous fungus of the genus *Trichoderma* (Reference Example 2) are taken as 100%.

### Description of Embodiments

Hereinafter, the present invention will be described in detail.

### <Filter aid and method of filtration treatment>

A filter aid is a substance that has one or more of the following functions: reducing filtration resistance, reducing clogging of the filter media, and improving the clarity of the filtrate. In general, filter aids are roughly divided into organic filter aids and inorganic filter aids. Organic filter aids (e.g., biodegradable fiber polymers such as cellulose and chitin) may be decomposed by the target of filtration (e.g., cellulase). Therefore, the present invention is characterized by using inorganic filter aid. The most representative type of inorganic filter aid is diatomaceous earth, which is produced as a fossil of diatoms, a type of phytoplankton. In addition to diatomaceous earth, perlite derived from lava erupted by volcanic activity is another example of the inorganic filter aid, although most perlite is used as a supplement to diatomaceous earth filter aid. Therefore, in the inorganic filter aid used in the present invention, the main ingredient is preferably diatomaceous earth and/or perlite as its active ingredient, the main ingredient is more preferably diatomaceous earth. Even more preferably, diatomaceous earth per se is used as the inorganic filter aid. Note that the phrase "mainly composed of" means more than 50 wt% of ingredients.

Diatomaceous earth differs from other algae in that diatoms are covered with porous, rigid cell walls made of amorphous hydrated silicic acid (SiO₂·nH₂O), which is called a silica shell and has numerous pores about 0.1 to 1 µm in diameter. These pores are macroscopic compared with the pore size of activated carbon or zeolite (0.2 to 20 nm), and do not have the same adsorption performance as activated carbon or zeolite (J. Soc. Powder Technol., Japan, 39, p114 - 121, 2002). Mined raw materials are refined through operations such as drying, classification, and calcination. Filter aids composed of diatomaceous earth are roughly divided into calcined products and flux-calcined products, but either type may be used as diatomaceous earth that is used as the main ingredient of the inorganic filter aid.

Commercially available diatomaceous earth can be used. Examples of calcined products include Radiolite (registered trademark) series manufactured by Showa Chemical Industry Co., Ltd. such as "Radiolite #100", "Radiolite #200", "Radiolite #300", and "Radiolite #500"; and Celite (trademark) series manufactured by Imerys such as Standard Super-Cel, "Celite 350", "Celite 505", "Celite 512", and "Celite 577". Among them, "Celite 512" is preferred. Examples of flux-calcined products include Radiolite (registered trademark) series such as "Radiolite #700", "Radiolite #800", "Radiolite #900", and "Radiolite #3000", and Celite (trademark) series manufactured by Imerys such as Hyflo Supercel: "Celite 281", "Celite 499", and "Celite 503". Among them, "Radiolite #700" is preferred.

The filter aid of the present invention is characterized in that it is a mixture of the inorganic filter aid and the filamentous fungal culture liquid mentioned above. In addition, the mixture which is the filter aid of the present invention is characterized in that it is a mixture prepared independently of the filtration treatment step. For example, when an inorganic filter aid is added during filtration treatment of the filamentous fungal culture liquid, a mixture of the inorganic filter aid and the filamentous fungal culture liquid is to be prepared as a liquid to be filtered, and such a mixture does not correspond to the filter aid of the present invention.

The filamentous fungus of the filamentous fungal culture liquid used in the filter aid of the present invention is not particularly limited, and examples thereof include filamentous fungi of the genus *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Piromyces, Pleurotus, Rhizopus, Schizophyllum, Talaromyces, Thermoascus, Thielavia, Tolypocladium, Trametes,* and *Trichoderma.* Specific examples of these filamentous fungi include *Acremonium, Aspergillus, Chrysosporium, Fusarium, Humicola, Myceliophthora, Neurospora, Penicillium, Piromyces, Talaromyces, Thermoascus, Thielavia,* and *Trichoderma.* The filamentous fungus of the genus *Trichoderma* or the genus *Talaromyces* is preferred.

The filamentous fungus of the genus *Trichoderma* used for preparing the filamentous fungal culture liquid may be a wild-type strain or variant of the filamentous fungus of the genus *Trichoderma* that has been improved so as to have high protein production capacity. Specific examples of the filamentous fungus of the genus *Trichoderma* include *Trichoderma reesei, Trichoderma viride, Trichoderma atroviride,* and *Trichoderma longibrachiatum.* Among them, *Trichoderma reesei* is preferred. Among them, *Trichoderma reesei* is preferred. Specific examples of *Trichoderma reesei* include *Trichoderma parareesei* (ATCC MYA-4777), which is an ancestor of *Trichoderma reesei,* and known variants derived from *Trichoderma reesei* such as QM6a strain (NBRC31326), QM9123 strain (ATCC24449), QM9414 strain (NBRC31329), PC-3-7 strain (ATCC66589), QM9123 strain (NBRC31327), RutC-30 strain (ATCC56765), CL-847 strain (Enzyme. Microbiol. Technol.,10, 341 - 346 (1988)), MCG77 strain (Biotechnol. Bioeng. Symp., 8, 89 (1978)), MCG80 strain (Biotechnol. Bioeng., 12, 451 - 459 (1982)) and derivatives thereof. Further, variants with mutations described in WO2019/188980, WO2019/230860, WO2020/027010, WO2020/045472, WO2020/045473, WO2020/075787, and WO2020/075788 originating from QM9414 strain can be used. Note that QM6a, QM9414, and QM9123 strains are available from NBRC (NITE Biological Resource Center), and PC-3-7 and RutC-30 strains are available from ATCC (American Type Culture Collection).

The filamentous fungus of the genus *Talaromyces* used for preparing the filamentous fungal culture liquid may be a wild-type strain or variant of the filamentous fungus of the genus *Talaromyces* that has been improved so as to have high protein production capacity. Specific examples of filamentous fungus of the genus *Talaromyces* are not particularly limited as long as they have the above-mentioned characteristics, but preferably include *Talaromyces marneffei, Talaromyces stipitatus, Talaromyces amestolkiae, Talaromyces atroroseus, Talaromyces verruculosus, Talaromyces cellulolyticus, Talaromyces pinophilus,* and *Talaromyces emersonii. Talaromyces cellulolyticus* is more preferred. Specific examples of *Talaromyces cellulolyticus* include known variants derived from *Talaromyces cellulolyticus* such as Y-94 strain (FERM BP-5826), TN strain (FERM BP-11452), C1 strain (FERM P-18508), CF-2612 strain (FERM BP-10848), and derivatives thereof.

The method of culturing a filamentous fungus is not particularly limited. For example, it is cultured by liquid culture using a centrifuge tube, a flask, a jar fermenter, a tank, or the like; or solid culture using a plate. The filamentous fungus of the genus *Trichoderma* is cultured preferably under aerobic conditions. Among these methods of culturing, submerged culture is preferred, which involves culturing in a flask, particularly a jar fermenter, or a tank, with aeration or stirring. The aeration rate is preferably about 0.1 to 2.0 vvm, more preferably 0.3 to 1.5 vvm, particularly preferably 0.5 to 1.0 vvm. The culture temperature is preferably 25 to 35°C, more preferably 25 to 31°C. The pH condition during culturing is preferably from pH 3.0 to 7.0, more preferably from pH 4.0 to 6.0. As for the culture time, culturing is performed until a recoverable amount of microorganism cells are accumulated under conditions that allow microorganism cells to grow or protein to be produced. The culture time is usually about 24 to 288 hours, more preferably 36 to 240 hours.

As the culture liquid of filamentous fungus, a culture liquid is used which has been cultured until a dry cell weight of the filamentous fungus reaches preferably 1 g/L or more, more preferably 3 g/L or more, still more preferably 5 g/L or more, and particularly preferably 10 g/L or more. The upper limit of the dry cell weight of the filamentous fungus after culturing of the filamentous fungus is not particularly limited as long as it does not impair the effects of the present invention, but is preferably 50 g/L, more preferably 40 g/L. The dry cell weight can be determined as follows. Two milliliters of the filamentous fungal culture liquid is placed in a 2 mL Eppendorf tube, centrifuged at 20,000 ×g at 4°C for 10 minutes in a centrifuge, and after the supernatant is discarded, washed with distilled water, then centrifuged again at 20,000 ×g at 4°C for 10 minutes in a centrifuge, and the recovered microorganism cell pellets are dried in a dry heat sterilizer at 105°C for 2 hours, and the weight (g-cell/2 mL) is measured using an electronic balance, and converted into the dry cell weight (g-cell/L).

The filamentous fungal culture liquid may be a culture liquid from which filamentous fungal cells have been removed, or a culture liquid containing filamentous fungal cells, but a culture liquid containing filamentous fungal cells is preferably used.

A mixing ratio between the inorganic filter aid and the filamentous fungal culture liquid is such that the concentration of the inorganic filter aid included in the filamentous fungal culture liquid is preferably 1 to 15% (w/v), more preferably 3 to 12% (w/v), still more preferably 5 to 10% (w/v). When the concentration is more than 15% (w/v), the fluidity of the liquid to be filtered decreases, which may deteriorate filterability.

The pH set when mixing the inorganic filter aid with the filamentous fungal culture liquid is preferably pH 2.0 to 7.0, more preferably pH 3.0 to 6.0. It is not preferable to use a filter aid mixed with pH set at 8.0 or more, because the desired effect cannot be obtained.

The time for mixing and incubating the inorganic filter aid and the filamentous fungal culture liquid is not particularly limited, but is preferably 1 hour or more, more preferably 6 hours or more, still more preferably 12 hours or more.

Examples of the method of filtration treatment that requires the use of the filter aid of the present invention include gravity filtration, vacuum filtration, and pressure filtration. When performing gravity filtration, for example, a sand bed filter, or a bag filter can be used. When performing vacuum filtration, devices such as a nutsche, a precoat filter (drum filter), or the like can be used. When performing pressure filtration, devices such as a candle filter, a leaf filter, a filter press, or a bag filter can be used. As the method of filtration treatment to which the filter aid of the present invention can be applied, vacuum filtration and pressure filtration are preferred, and pressure filtration are more preferred. In addition, as the device used to perform pressure filtration, a leaf filter and a filter press are preferred, and a filter press is more preferred. The filter press may be of vertical or horizontal type.

An exemplary method of filtration treatment using the filter aid of the present invention is a method in which the filter aid of the present invention is added to the liquid to be filtered (body feed), and then filtered according to the method of filtration treatment mentioned above. In this method, cake formed by filtering the liquid to be filtered containing the filter aid includes a mixture of suspended matters and the filter aid, and has high void ratio and low filtration resistance. In this method, it is preferable that the filter aid of the present invention be preferably 1 to 15% (w/v), more preferably 3 to 12% (w/v), and still more preferably 5 to 10% (w/v) as an equivalent amount of the inorganic filter aid contained in the filter aid, with respect to the amount of the liquid to be filtered. When the amount as the equivalent amount of inorganic filtration aid is more than 15% (w/v), the fluidity of the liquid to be filtered may decrease, which may deteriorate filterability.

An exemplary method of filtration treatment to which the filter aid of the present invention is applied may be a method in which the filter aid of the present invention is previously applied (precoated) onto the surface of a filter cloth, then the liquid to be filtered may be passed from the coated surface side for filtration. The filter aid of the present invention to be precoated preferably contains filamentous fungal cells. For example, a 2 to 6 mm thick layer (precoated layer) composed of the filter aid is formed on the filter cloth to capture suspended matters that causes filtration blockages and to facilitate the operation of peeling off the cake after filtration operation from the filter medium. The type of the filter cloth to be precoated is not particularly specified as long as the filter cloth can be used in the method of filtration treatment mentioned above, and may be, for example, a plain dutch weave, a twilled dutch weave, or a reverse twilled dutch weave. The filter cloth can be purchased from Nakao Filter Media Corp., Daiki Ataka Engineering Co., Ltd. and the like.

The target of the filtration treatment to which the filter aid of the present invention can be applied is not particularly limited, but can be suitably used in a filtration treatment to separate microorganism cells from a culture liquid of microorganism. Specific examples include a filtration treatment to separate microorganism cells from a culture liquid of microorganism capable of producing cellulase to recover cellulase. Particularly, in a filtration treatment for recovering cellulase having β-xylosidase activity by filtering microorganism cells from a culture liquid of microorganism capable of producing cellulase having β-xylosidase activity, the filtration treatment using the filter aid of the present invention can recover cellulase without impairing the β-xylosidase activity.

### <Method of manufacturing cellulase>

Cellulases include a variety of hydrolases, such as enzymes with degrading activity against xylan, cellulose, hemicellulose. Specific examples include cellobiohydrolase (EC 3.2.1.91), which produces cellobiose by hydrolyzing cellulose chains; endoglucanase (EC 3.2.1.4), which hydrolyzes from the central portion of cellulose chains; β-glucosidase (EC 3.2.1.21), which hydrolyzes cellooligosaccharides and cellobiose; xylanase (EC 3.2.1.8), which is characterized by acting on hemicellulose; and β-xylosidase (EC 3.2.1.37), which hydrolyzes xylooligosaccharides. Furthermore, cellulases secreted by the filamentous fungus of the genus *Talaromyces,* which will be described later, include pectinase and galactanase as enzymes that break down polysaccharides such as pectin and galactan. For example, as the commercially available cellulase preparation derived from the filamentous fungus of the genus *Talaromyces,* "Acremonium Cellulase" (Meiji Seika Pharma), has high pectinase activity and galactanase activity in addition to cellulase activity.

The filter aid of the present invention is characterized in that it produces cellulase by culturing a microorganism capable of producing cellulase, allowing cellulase to accumulate in the culture liquid, and performing solid-liquid separation by filtration treatment using the filter aid of the present invention.

Examples of microorganisms capable of producing cellulase include bacteria, yeasts, and filamentous fungi. Among them, filamentous fungi are preferred. Examples of the filamentous fungi include filamentous fungi of the genera *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Piromyces, Pleurotus, Rhizopus, Schizophyllum, Talaromyces, Thermoascus, Thielavia, Tolypocladium, Trametes,* and *Trichoderma.* Among these filamentous fungi, those selected from the group consisting of the genera *Acremonium, Aspergillus, Chrysosporium, Fusarium, Humicola, Myceliophthora, Neurospora, Penicillium, Piromyces, Talaromyces, Thermoascus, Thielavia,* and *Trichoderma* are preferred. Furthermore, from the viewpoints of productivity of cellulase and the performance of the resulting cellulase to saccharify biomass, filamentous fungi of the genera *Trichoderma* and *Talaromyces* are more preferred. Specific examples of the filamentous fungi of the genera *Trichoderma* and *Talaromyces* include the above-mentioned filamentous fungi of the genera *Trichoderma* and *Talaromyces,* which are used to prepare the filter aid of the present invention.

The filter aid of the present invention is preferably used in solid-liquid separation from the culture liquid of microorganisms capable of producing cellulases having β-xylosidase activity, preferably microorganisms capable of producing cellulases having pectinase activity and/or galactanase activity for recovering cellulase having these enzymatic activities.

Examples of microorganisms capable of producing cellulase having β-xylosidase activity include bacteria, yeasts, and filamentous fungi. Among them, filamentous fungi are preferred. Examples of the filamentous fungi include filamentous fungi of the genera *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Piromyces, Pleurotus, Rhizopus, Schizophyllum, Talaromyces, Thermoascus, Thielavia, Tolypocladium, Trametes,* and *Trichoderma.* Among these filamentous fungi, those selected from the group consisting of the genera *Acremonium, Aspergillus, Chrysosporium, Fusarium, Humicola, Myceliophthora, Neurospora, Penicillium, Piromyces, Talaromyces, Thermoascus, Thielavia,* and *Trichoderma* are preferred, and filamentous fungus of the genus *Trichoderma* is more preferred. Specific examples of the filamentous fungi of the genera *Trichoderma* and *Talaromyces* include the above-mentioned filamentous fungi of the genera *Trichoderma* and *Talaromyces,* which are used to prepare the filter aid of the present invention.

A medium commonly used in the field of cellulase production is used to culture microorganisms capable of producing cellulase. Furthermore, the amount of cellulase produced can be improved by culturing in a medium containing a cellulase inducer during culturing. Examples of the cellulase inducer include degradative intermediates of cellulose, including lactose, sophorose, gentibiose and cellobiose; biomass containing crystalline cellulose, xylan, and cellulose and xylan. Specific examples of biomass containing cellulose and xylan include pulp, bagasse, cassava pulp, switchgrass, napier grass, erianthus, corn stover, corn cob, corn hull, rice straw, wheat straw, beet pulp, eucalyptus, oak, and white birch. Pulp or corn hulls are preferred, and corn hulls are more preferably used. An example of a cellulase inducer made from corn hulls is ground corn hulls described in WO 2021/235419.

In addition, a pretreated biomass containing cellulose and xylan is also preferably used as a cellulase inducer. As the pretreatment method, known techniques such as acid treatment, sulfuric acid treatment, dilute sulfuric acid treatment, alkali treatment, hydrothermal treatment, subcritical treatment, fine pulverization treatment, steaming treatment, and pulverization treatment can be used.

Furthermore, in order to further increase the cellulase accumulation concentration, liquid sugar or biomass may be added during the culture. Specific examples of liquid sugars to be added include degradative intermediates of cellulose including glucose, fructose, sucrose, maltose, lactose, sophorose, gentibiose, and cellobiose. Specific examples of biomass include biomasses containing crystalline cellulose, xylan, and cellulose and xylan. Specific examples of biomass containing cellulose and xylan include the above-mentioned biomass. When added to a fermenter, liquid sugar is preferred from the viewpoint of ease of handling, and glucose and lactose are particularly preferred. The timing for starting the addition of the liquid sugar to be used is preferably within 144 hours from the start of culturing, more preferably within 72 hours from the start of culturing, and particularly preferably within 24 hours from the start of culturing. The sugar may be added once, multiple times, or continuously.

The method of culturing microorganisms capable of producing cellulase to accumulate cellulase in a culture liquid is not particularly limited. For example, they are cultured by liquid culture using a centrifuge tube, a flask, a jar fermenter, a tank, or the like; and solid culture using a plate. The aeration conditions for microorganisms capable of producing cellulase may be those suitable for the microorganisms to be cultured, but when a filamentous fungus of the genus *Trichoderma* is used as the microorganisms, it is cultured preferably under aerobic conditions. Among these culturing methods, submerged culture is preferred, which involves culturing in a flask, particularly a jar fermenter, or a tank, with aeration or stirring. The aeration rate is preferably about 0.1 to 2.0 vvm, more preferably 0.3 to 1.5 vvm, particularly preferably 0.5 to 1.0 vvm. The culture temperature is preferably about 25 to 35°C, more preferably 25 to 31°C. The pH condition during culturing is preferably from pH 3.0 to 7.0, more preferably from pH 4.0 to 6.0. As for the culture time, culturing is performed until recoverable amount of microorganism cells are accumulated under conditions that allow microorganism cells to grow or protein to be produced. The culture time is usually about 24 to 288 hours, more preferably 36 to 240 hours.

Prior to the above-mentioned culturing, it is preferable to preculture a microorganism capable of producing cellulase, and then subject the preculture liquid to the above-mentioned culturing. As carbon sources used in the preculturing, a sugar that does not induce cellulase, such as glucose, fructose, sucrose, and maltose, or a cellulase inducer, such as biomass containing cellobiose, crystalline cellulose, xylan, or cellulose and xylan. However, from the viewpoint of culture operations such as ease of assimilation and transportability to the main culture liquid, glucose is preferably used. The culture conditions are preferably applied to the culturing mentioned above. When the filamentous fungus of the genus *Trichoderma* or the genus *Talaromyces* is used as the microorganism capable of producing cellulase, a portion of a preculture liquid of the filamentous fungus of the genus *Trichoderma* or the genus *Talaromyces* can be used as the culture liquid of the filamentous fungus of the genus *Trichoderma* or the genus *Talaromyces,* which is a main ingredient of the filter aid of the present invention.

The cellulase-containing culture liquid obtained by the above mentioned method is preferably reduced in the microorganism cell fraction prior to filtration treatment. Reducing microorganism cell fraction in advance prevents a filter chamber of the device used in the filtration treatment from becoming filled with solid matter. Centrifugation is a preferred method to reduce microorganism cell fraction. Examples of device used for centrifugation include bottom discharge type, continuous type, and separation plate type (DeLaval type) devices. The centrifugation conditions are not particularly limited as long as they allow microorganism cells to be precipitated and removed.

In the present invention, the cellulase-containing culture liquid is filtered together with the filtration aid of the invention to separate the microorganism cells and recover cellulase. Furthermore, the present invention is characterized in that the filter aid is prepared independently of the filtration treatment step. For example, in a filtration treatment step to separate cellulase from a cellulase-containing culture liquid of filamentous fungus of the genus *Trichoderma,* when diatomaceous earth is body-fed as a filter aid or when a filter cloth is precoated with diatomaceous earth as a filter aid, a liquid to be filtered is a mixture of diatomaceous earth and a cellulase-containing culture liquid of filamentous fungus of the genus *Trichoderma.* Such a mixture does not correspond to the filter aid of the present invention, and the embodiment does not correspond to "preparing the filter aid of the present invention independently of the manufacturing step of cellulase". The method of filtration treatment of the cellulase-containing culture liquid to which the filter aid of the present invention is applied is as described above, and may be a filtration treatment in which a filter aid is body-fed, a filtration treatment in which a filter cloth is precoated with a filter aid, or both.

Cellulase from which microorganism cells have been separated by filtration may be used per se as a crude enzyme liquid, or it may be formulated by known methods before use.

The cellulase obtained in the present invention can be widely used for hydrolysis of cellulose-containing biomass. Cellulose-containing biomass contains at least cellulose or hemicellulose, and specific examples thereof include pulp, bagasse, cassava pulp, switchgrass, napier grass, erianthus, corn stover, corn cob, corn hull, rice straw, wheat straw, beet pulp, eucalyptus, oak, and white birch. These cellulose-containing biomasses contain impurities such as lignin which is an aromatic polymer compound and hemicellulose, and a cellulose-containing biomass which has been pretreated using an acid, alkali and pressurized hot water, etc. to partially decompose lignin and hemicellulose may be used as cellulose. The hydrolysis conditions may be set to the optimum reaction conditions for the cellulase derived from a filamentous fungus, and preferably the treatment temperature is 40 to 60°C, the treatment pH is 3 to 7, and the solid content in the cellulose-containing biomass is 0.1 to 30%. By setting the reaction conditions within the optimum range described above, the efficiency of biomass hydrolysis can be maximized. The hydrolysis may be performed in a batchwise manner or in a continuous manner. The hydrolysate obtained by such an enzymatic treatment contains monosaccharide ingredients such as glucose and xylose, and thus can be used as raw sugar for fermentation to produce various chemical products.

When using the cellulase obtained in the present invention, enzyme ingredients with other functions such as oxidase, reductase, hydrolyase, transferase, and isomerase may be added to provide an enzyme preparation. In particular, enzyme ingredients derived from any organism, not limited to filamentous fungi, and enzyme ingredients produced by gene recombination may be used as such enzyme ingredients with other functions.

### Examples

Hereinafter, the present invention will be specifically described with reference to Examples, but the present invention is not intended to be limited thereto.

### <Reference example 1> Preparation of a culture liquid of filamentous fungus

Spores of *Trichoderma reesei* PC-3-7 (ATCC 66589) (hereinafter, also referred simply as "filamentous fungus of the genus *Trichoderma"*) were diluted with physiological saline to a concentration of 1.0 × 10⁷ /mL. Two 1-L baffled flasks containing 250mL of the medium shown in Table 1 were prepared, inoculated with 2.5mL of diluted spore solution, and cultured in a shaking incubator for 72 hours at 28°C at 120 rpm. One of these flasks was inoculated in the main culture liquid of Reference Example 2, and the other was continued to be cultured until the end of the main culture in order to use it for the preparation of the filter aid of Reference Example 2.

After culturing was completed, 2 mL of the culture liquid of filamentous fungus of the genus *Trichoderma* was taken in a 2-mL volume Eppendorf tube, centrifuged at 20,000 ×g at 4°C for 10 minutes in a centrifuge, and the supernatant was discarded. After washing with distilled water, centrifugation was performed again in a centrifuge at 20,000 ×g at 4°C for 10 minutes to recover microorganism cell pellets. The recovered microorganism cell pellets were dried in a dry heat sterilizer at 105°C for 2 hours, and its weight (g-cell/2 mL) was measured using an electronic balance, and the dry cell weight (g-cell/L) of the culture liquid of filamentous fungus of the genus *Trichoderma* was determined as 1.5 g/L.

When *Talaromyces cellulolyticus* C1 strain (FERM P-18508) (hereinafter, also referred to simply as "filamentous fungus of the genus *Talaromyces*") was used as the filamentous fungus, the culturing was performed in the same manner. In the same manner as for the culture liquid of filamentous fungus of the genus *Trichoderma,* the dry cell weight (g-cell/L) of the culture liquid of filamentous fungus of the genus *Talaromyces* was determined as 34.7 g/L.

**[Table 1]**

| | |
|---|---|
| Glucose | 20 g |
| 5 × Mandel's solution * | 200 mL |
| 10 × Ammonium tartrate solution ** | 100 mL |
| Corn steep liquor | 50g |
| Trace element solution*** | 1 mL |
| Tween 80 | 0.5 mL |
| PE-M | 1 mL (per L) |

| | |
|---|---|
| *5 × Mandel's solution has the following composition: 7 g/L (NH4)₂SO₄ 10 g/L KH₂PO₄ 2 g/L CaCl₂·2H₂O 1.5 g/L MgSO₄·7H₂O **10 × Ammonium tartrate solution contains 92 g/L of ammonium tartrate ***Trace element solution has the following composition: 0.3 g/L H₃BO₃ 1.3 g/L (NH₄)6Mo₇O₂₄·4H_{2O} 5 g/L FeCl₃·6H₂O 2 g/L CuSO₄·5H₂O 0.4 g/L MnCl₂·4H₂O 10 g/L ZnCl₂ | |

### <Reference example 2> Preparation of a crude enzyme liquid derived from filamentous fungus

When *Trichoderma reesei* strain PC-3-7 was used as the filamentous fungus, 2.5 L of the medium for cellulase production listed in Table 2 was placed in a 5 L jar fermenter (manufactured by BIOTT CO., LTD.) and autoclaved at 121°C for 40 minutes. 250 mL of the culture liquid of *Trichoderma reesei* PC-3-7 of Reference Example 1 as a preculture liquid was inoculated into a medium for cellulase production (inoculum amount 10% v/v). As for culture conditions, after the culture liquid prepared in Reference Example 1 was inoculated into the main culture medium, submerged culture was started at 28°C, at 700 rpm, and at the aeration rate of 1 vvm, while controlling pH to 5.0. After 10 hours of culturing, a sugar liquid containing glucose and lactose shown in Table 3 was added at a rate of 225 mL/day, and culturing was performed while adjusting the sugar liquid feed so that the dissolved oxygen level was 20%.

When *Talaromyces cellulolyticus* C1 strain was used as the filamentous fungus, culturing was performed under the same conditions as those for the *Trichoderma reesei* PC-3-7 strain described above, except for cellulase inducer (lactose), culture liquid temperature (30°C), pH condition (pH 4.0), and fed-batch condition (without fed-batch).

**[Table 2]**

| | |
|---|---|
| Ground corn hull | 100 g |
| 5 × Mandel's solution* | 200 mL |
| Corn steep liquor | 25 g |
| Trace element solution *** | 1 mL |
| PE-M | 1 mL (per L) |

| | |
|---|---|
| * same as in Table 1 *** same as in Table 1 | |

**[Table 3]**

| | |
|---|---|
| Glucose | 500 g |
| Lactose | 125 g |
| (NH4)₂SO₄ | 8.75 g |
| KH₂PO₄ | 5.00 g (per L) |

After the culturing was completed, 80mL of the culture liquid was collected and dispensed equally into two 50-mL centrifuge tubes (manufactured by Corning Inc.). The centrifuge tube was centrifuged at 3,000 x g at 4°C for 10 minutes to remove the microorganism cells, and the crude enzyme liquid derived from the filamentous fungus of the genus *Trichoderma* and the crude enzyme liquid derived from the filamentous fungus of the genus *Talaromyces* were obtained by decantation. For these crude enzyme liquids, protein concentration and specific activity of β-xylosidase were measured according to the methods described in Reference Examples 4 and 5 below, and for the crude enzyme liquid derived from the filamentous fungus of the genus *Talaromyces,* specific activity of pectinase and specific activity of galactanase were measured according to the methods described in Reference Examples 6 and 7 below, and these values were taken as 100% and compared with the values in the Examples and Comparative Examples described below.

### <Reference Example 3> Preparation of alkali-treated bagasse

9.3 g of caustic soda was mixed with 100 g of dry bagasse, and the mixture was reacted at 121°C for 30 minutes to prepare an alkali-treated bagasse.

### <Reference Example 4> Conditions for measurement of protein concentration

Reagent for measuring protein concentration: Quick Start (trademark)
Bradford 1 × Dye Reagent (manufactured by Bio-Rad Laboratories, Inc.)
Reagent for measuring protein concentration: 250 µL
Diluted crude enzyme liquid: 5 µL
Measurement temperature: Room temperature
Reaction time: 5 minutes
Absorbance: 595 nm
Standard: BSA.

### <Reference Example 5> Conditions for measuring specific activity of β-xylosidase

Substrate: p-Nitrophenyl-β-xylopyranoside (manufactured by Sigma Aldrich Japan)
Reaction liquid: 90 µL of 50 mM acetate buffer containing 1 mM p-nitrophenyl-β-xylopyranoside
Diluted crude enzyme liquid: 10 µL
Reaction temperature: 30°C
Reaction time: 10 minutes
Reaction terminator: 2 M Sodium carbonate, 10 µL
Absorbance: 405 nm
Enzyme reaction: A diluted enzyme liquid was mixed with a substrate cooled to 4°C on a 96-well PCR plate, and incubated at 30°C for 10 minutes using a thermal cycler. Thereafter, the reaction liquid was promptly cooled to 4°C, and 10 µL of 2 M sodium carbonate was added to terminate the enzyme reaction.

Quantification of activity: The absorbance of the reaction liquid at 405 nm was measured with a microplate reader, and the amount of p-nitrophenol liberated in the reaction liquid was calculated from the values on a calibration curve based on a standard. One U of activity was defined as the amount of enzyme that liberates 1 µmol of p-nitrophenol per minute, and the β-xylosidase activity was calculated from the amount of p-nitrophenol contained in the diluted enzyme liquid. The activity per mg of protein was calculated by dividing the β-xylosidase activity contained in the diluted enzyme liquid by the protein concentration of the diluted enzyme liquid.

### <Reference Example 6> Conditions for measuring specific activity of pectinase

Substrate: 90 µL of 100mM sodium acetate (pH5.0) containing 5.5 g/L polygalacturonic acid
Diluted enzyme liquid: 10 µL (diluted to protein concentration of 0.25 g/L)
Standard: 0.25 to 4 g/L Galacturonic acid
Chromogenic reagent: DNS reagent (5 g/L 3,5-dinitrosalicylic acid (DNS) and 300 g/L sodium tartrate dissolved in 0.4 M sodium hydroxide)
Enzyme reaction: A diluted enzyme liquid was mixed with a substrate cooled to 4°C on a 96-well PCR plate, and incubated at 50°C for 10 minutes using a thermal cycler.

Thereafter, the reaction liquid was promptly cooled to 4°C, and 10 µL of 1 M sodium hydroxide was added to terminate the enzyme reaction.

Chromogenic reaction: 40 µL of enzyme reaction liquid and 80 µL of DNS reagent were mixed on a new 96-well PCR plate, and heated at 95°C for 5 minutes using a thermal cycler. The chromogenic liquid was cooled to room temperature, mixed with 120 µL of water, and then 180 µL portion was transferred to a new 96-well microplate. Quantification of activity: The absorbance of the chromogenic liquid at 540 nm was measured with a microplate reader, and the amount of reducing sugar liberated in the reaction liquid was calculated from the values on a calibration curve based on a standard. One U of activity was defined as the amount of enzyme that liberates 1 µmol of reducing sugar per minute, and the pectinase activity was calculated from the amount of reducing sugar contained in the diluted enzyme liquid. The activity per mg of protein was calculated by dividing the pectinase activity contained in the diluted enzyme liquid by the protein concentration of the diluted enzyme liquid.

### <Reference Example 7> Conditions for measuring specific activity of galactanase

Substrate: 90 µL of 100mM sodium acetate (pH5.0) containing 5.5 g/L potato-derived galactan
Diluted enzyme liquid :10 µL (diluted to protein concentration of 0.25 g/L)
Standard: 0.25 to 4g/L Galactose
Chromogenic reagent: DNS reagent
Enzyme reaction: A diluted enzyme liquid was mixed with a substrate cooled to 4°C on a 96-well PCR plate, and incubated at 50°C for 10 minutes using a thermal cycler.

Thereafter, the reaction liquid was promptly cooled to 4°C, and 10 µL of 1 M sodium hydroxide was added to terminate the enzyme reaction.

Chromogenic reaction: 40 µL of enzyme reaction liquid and 80 µL of DNS reagent were mixed on a new 96-well PCR plate, and heated at 95°C for 5 minutes using a thermal cycler. The chromogenic liquid was cooled to room temperature, mixed with 120 µL of water, and 180 µL portion was transferred to a new 96-well microplate.

Quantification of activity: The absorbance of the chromogenic liquid at 540 nm was measured using a microplate reader, and the amount of reducing sugar liberated in the reaction liquid was calculated from the values on a calibration curve based on a standard. One U of activity was defined as the amount of enzyme that liberates 1 µmol of reducing sugar per minute, and the galactanase activity was calculated from the amount of reducing sugar contained in the diluted enzyme liquid. The activity per mg of protein was calculated by dividing the galactanase activity contained in the diluted enzyme liquid by the protein concentration of the diluted enzyme liquid.

### <Reference Example 8> Measurement of sugar concentration

A solution was prepared by adding 20 µL of 1N NaOH as a reaction terminator liquid to 180µL of the crude enzyme liquid, and xylose was quantitatively analyzed using ACQUITY UPLC (Waters, Milford, MA, USA) under the following conditions to measure the concentration.
Column: ACQUITY UPLC BEH Amide column (2.1 × 100 mm, 1.7 µm, manufactured by Waters Corporation)
Mobile phase: 80% (v/v) acetonitrile and 0.2% (v/v) trimethylamine (TEA) mixed solution
Flow rate: eluted at 0.3 mL/min with a gradient of acetonitrile from 80% to 75% Temperature: 50°C
Detection method: ELSD (evaporative light scattering detector system, manufactured by Waters Corporation).

### <Comparative Example 1> Solid-liquid separation of a crude enzyme liquid derived from filamentous fungus of the genus Trichoderma using diatomaceous earth as a filter aid (precoat agent)

Diatomaceous earth "Celite 512" was added to distilled water to a concentration of 10% (w/v), and subjected to shaking culture at room temperature at 120 rpm for an hour to obtain a filter aid for precoating. Ten milliliters of this filter aid was added dropwise onto a filter cloth of a polysulfone holder, and then suction filtered with a vacuum pump to form a precoat layer of the filter aid. The eluted water was discarded.

Next, 20mL of crude enzyme liquid derived from the filamentous fungus of the genus *Trichoderma* prepared in Reference Example 2 was gently added dropwise onto a polysulfone holder on which a precoat layer had been formed, and then subjected to suction filtration with a vacuum pump. The resulting filtrate was recovered, and the protein concentration and specific activity of β-xylosidase were measured by the methods described in Reference Examples 4 and 5.

As shown in Table 4, the protein concentration was slightly decreased after suction filtration compared to that before the suction filtration, which is considered to be due to dilution by the remaining water at the time of formation of precoat. Further, specific activity of β-xylosidase was 53.9% compared to that of the unfiltered crude enzyme liquid derived from the filamentous fungus of the genus *Trichoderma* (Reference Example 2).

### <Example 1> Solid-liquid separation of a crude enzyme liquid derived from the filamentous fungus of the genus Trichoderma using a mixture of diatomaceous earth and a culture liquid containing cells of filamentous fungus of the genus Trichoderma as a filter aid (precoat agent)

The entire amount of the culture liquid of filamentous fungus of the genus *Trichoderma* prepared by the method described in Reference Example 1 was centrifuged at 3,000 ×g and 4°C for 10 minutes to separate the supernatant and the microorganism cells. The microorganism cell fraction was recovered, suspended in distilled water, and centrifuged at 3,000 ×g at 4°C for 10 minutes, and the microorganism cell fraction was washed. After the washed microorganism cell fraction was suspended in distilled water in an amount equivalent to that of the separated supernatant fraction, diatomaceous earth "Celite 512" was added to the suspension to a concentration of 10% (w/v), and subjected to shaking culture at room temperature at 120 rpm for an hour to obtain a filter aid for precoating. The crude enzyme liquid derived from the filamentous fungus of the genus *Trichoderma,* prepared in Reference Example 2 in the same manner as in Comparative Example 1 except that this filter aid was used, was subjected to suction filtration, the filtrate was recovered, and the protein concentration and specific activity of β-xylosidase of the recovered filtrate were measured by the methods described in Reference Examples 4 and 5.

As shown in Table 4, the protein concentration was slightly decreased after suction filtration compared to that before the suction filtration, which is considered to be due to dilution by the remaining water at the time of formation of precoat. On the other hand, specific activity of β-xylosidase was 74.7% compared to that of the unfiltered crude enzyme liquid derived from the filamentous fungus of the genus *Trichoderma* (Reference Example 2).

### <Example 2> Solid-liquid separation of a crude enzyme liquid of cellulase using a mixture of diatomaceous earth and a supernatant of the culture liquid of filamentous fungus of the genus Trichoderma as a filter aid (precoat agent)

The entire amount of the culture liquid of filamentous fungus of the genus *Trichoderma* prepared by the method described in Reference Example 1 was centrifuged at 3,000 ×g at 4°C for 10 minutes to separate the supernatant and the microorganism cells. Diatomaceous earth "Celite 512" was added to the resulting supernatant to a concentration of 10% (w/v), and subjected to shaking culture at room temperature at 120 rpm for an hour to obtain a filter aid for precoating. The crude enzyme liquid derived from the filamentous fungus of the genus *Trichoderma* prepared in Reference Example 2 was subjected to suction filtration to recover a filtrate in the same manner as in Comparative Example 1 except that this filter aid was used, and the protein concentration and specific activity of β-xylosidase of the recovered filtrate were measured by the methods described in Reference Examples 4 and 5.

As shown in Table 4, the protein concentration was slightly decreased after suction filtration compared to that before the suction filtration, which is considered to be due to dilution by the remaining water at the time of formation of precoat. On the other hand, specific activity of β-xylosidase was 68.1% compared to that of the unfiltered crude enzyme liquid derived from the filamentous fungus of the genus *Trichoderma* (Reference Example 2).

### <Comparative Example 2> Solid-liquid separation of a crude enzyme liquid derived from the filamentous fungus of the genus Trichoderma using diatomaceous earth as a filter aid (precoat agent and body feed agent)

Diatomaceous earth "Celite 512" was added to distilled water to a concentration of 10% (w/v), and subjected to shaking culture at room temperature at 120 rpm for an hour to obtain a filter aid for precoating and body feeding. Ten milliliters of this filter aid was added dropwise onto a filter cloth of a polysulfone holder, and then suction filtered with a vacuum pump to form a precoat layer of the filter aid. The eluted water was discarded.

Next, 10 mL of the filter aid mentioned above was added (body feed) to 10 mL of crude enzyme liquid derived from the filamentous fungus of the genus *Trichoderma* prepared in Reference Example 2, gently added dropwise onto a polysulfone holder on which a precoat layer had been formed, subjected to suction filtration with a vacuum pump to recover a filtrate, and the protein concentration and specific activity of β-xylosidase of the recovered filtrate were measured by the methods described in Reference Examples 4 and 5.

As shown in Table 4 and FIG. 1, the protein concentration was slightly decreased after suction filtration compared to that before the suction filtration, which is considered to be due to dilution by the remaining water at the time of formation of precoat. Further, specific activity of β-xylosidase was 22.5% compared to that of the unfiltered crude enzyme liquid derived from the filamentous fungus of the genus *Trichoderma* (Reference Example 2).

### <Comparative Example 3> Solid-liquid separation of a crude enzyme liquid derived from the filamentous fungus of the genus Trichoderma using a mixture of diatomaceous earth and a microorganism cell fraction of the culture liquid of filamentous fungus of the genus Trichoderma as a filter aid (precoat agent and body feed agent)

The entire amount of the culture liquid of filamentous fungus of the genus *Trichoderma* prepared by the method described in Reference Example 1 was centrifuged at 3,000 ×g at 4°C for 10 minutes to separate the supernatant and the microorganism cells. The microorganism cell fraction was recovered, suspended in distilled water, and centrifuged at 3,000 ×g at 4°C for 10 minutes, and the microorganism cell fraction was washed. After the washed microorganism cell fraction was suspended in distilled water in an amount equivalent to that of the separated supernatant fraction, diatomaceous earth "Celite 512" was added to the suspension to a concentration of 10% (w/v), and subjected to shaking culture at room temperature, 120 rpm for an hour to obtain a filter aid for precoating and body feeding. The crude enzyme liquid derived from the filamentous fungus of the genus *Trichoderma* was subjected to suction filtration to recover a filtrate in the same manner as in Comparative Example 2 except that this filter aid was used, and the protein concentration and specific activity of β-xylosidase of the recovered filtrate were measured by the methods described in Reference Examples 4 and 5.

As shown in Table 4, the protein concentration was slightly decreased after suction filtration compared to that before the suction filtration, which is considered to be due to dilution by the remaining water at the time of formation of precoat. On the other hand, specific activity of β-xylosidase was 26.2% compared to that of the unfiltered crude enzyme liquid derived from the filamentous fungus of the genus *Trichoderma* (Reference Example 2), which was found to be equivalent to that of Comparative Example 2.

### <Example 3> Solid-liquid separation of a crude enzyme liquid derived from the filamentous fungus of the genus Trichoderma using a mixture of diatomaceous earth and a culture liquid containing cells of the filamentous fungus of the genus Trichoderma as a filter aid (precoat agent and body feed agent)

Diatomaceous earth "Celite 512" was added to the entire amount of the culture liquid of filamentous fungus of the genus *Trichoderma* prepared by the method described in Reference Example 1 to a concentration of 10% (w/v), and subjected to shaking culture at room temperature, at 120 rpm for an hour to obtain a filter aid for precoating and body feeding. The crude enzyme liquid derived from the filamentous fungus of the genus *Trichoderma* was subjected to suction filtration to recover a filtrate in the same manner as in Comparative Example 2 except that this filter aid was used, and the protein concentration and specific activity of β-xylosidase of the recovered filtrate were measured by the methods described in Reference Examples 4 and 5.

As shown in Table 4 and FIG. 1, the protein concentration was slightly decreased after suction filtration compared to that before the suction filtration, which is considered to be due to dilution by the remaining water at the time of formation of precoat. On the other hand, specific activity of β-xylosidase was 88.4% compared to that of the unfiltered crude enzyme liquid derived from the filamentous fungus of the genus *Trichoderma* (Reference Example 2).

### <Example 4> Solid-liquid separation of a crude enzyme liquid derived from the filamentous fungus of the genus Trichoderma using a mixture of diatomaceous earth and the supernatant of the culture liquid of filamentous fungus of the genus Trichoderma as a filter aid (precoat agent and body feed agent)

The entire amount of the culture liquid of filamentous fungus of the genus *Trichoderma* prepared by the method described in Reference Example 1 was centrifuged at 3,000 ×g at 4°C for 10 minutes to separate the supernatant and the microorganism cells. Thereafter, a mixture of diatomaceous earth "Celite 512" and a supernatant fraction was prepared. The crude enzyme liquid derived from the filamentous fungus of the genus *Trichoderma* was subjected to suction filtration to recover a filtrate in the same manner as in Comparative Example 2 except that this mixture was used as a filter aid for precoating and body feeding, and the protein concentration and specific activity of β-xylosidase of the recovered filtrate were measured by the methods described in Reference Examples 5 and 6.

As shown in Table 4, the protein concentration was slightly decreased after suction filtration compared to that before the suction filtration, which is considered to be due to dilution by the remaining water at the time of formation of precoat. On the other hand, specific activity of β-xylosidase was 67.8% compared to that of the unfiltered crude enzyme liquid derived from the filamentous fungus of the genus *Trichoderma* (Reference Example 2).

### <Example 5> Solid-liquid separation of a crude enzyme liquid derived from the filamentous fungus of the genus Talaromyces using a mixture of diatomaceous earth and a culture liquid containing cells of the filamentous fungus of the genus Trichoderma as a filter aid (precoat agent and body feed agent)

Diatomaceous earth "Celite 512" was added to the entire amount of the culture liquid of filamentous fungus of the genus *Talaromyces* prepared by the method described in Reference Example 1 to a concentration of 10% (w/v), and subjected to shaking culture at room temperature, at 120 rpm for an hour to obtain a filter aid for precoating and body feeding. The crude enzyme liquid derived from the filamentous fungus of the genus *Trichoderma* was subjected to suction filtration to recover a filtrate in the same manner as in Comparative Example 2 except that this filter aid was used, and the protein concentration and specific activity of β-xylosidase of the recovered filtrate were measured by the methods described in Reference Examples 4 and 5.

As shown in Table 4, the protein concentration was slightly decreased after suction filtration compared to that before the suction filtration, which is considered to be due to dilution by the remaining water at the time of formation of precoat. On the other hand, specific activity of β-xylosidase was 81.2% compared to that of the unfiltered crude enzyme liquid derived from the filamentous fungus of the genus *Trichoderma* (Reference Example 2).

### <Comparative Example 4> Solid-liquid separation of a crude enzyme liquid derived from the filamentous fungus of the genus Talaromyces using diatomaceous earth as a filter aid (precoat agent and body feed agent)

Diatomaceous earth "Celite 512" was added to distilled water to a concentration of 10% (w/v), and subjected to shaking culture at room temperature at 120 rpm for an hour to obtain a filter aid for precoating and body feeding. Ten milliliters of this filter aid was added dropwise onto a filter cloth of a polysulfone holder, and then suction filtered with a vacuum pump to form a precoat layer of the filter aid. The separated water was discarded.

Next, 10 mL of the filter aid mentioned above was added (body feed) to 10 mL of crude enzyme liquid derived from the filamentous fungus of the genus *Talaromyces* prepared in Reference Example 2, gently added dropwise onto a polysulfone holder on which a precoat layer had been formed, subjected to suction filtration with a vacuum pump to recover a filtrate, and the protein concentration, specific activity of β-xylosidase, specific activity of pectinase, and specific activity of galactanase of the recovered filtrate were measured by the methods described in Reference Examples 4 to 7.

As shown in Table 4, specific activity of β-xylosidase decreased to 18.8% compared to that of the unfiltered crude enzyme liquid derived from the filamentous fungus of the genus *Talaromyces* (Reference Example 2). Further, specific activity of pectinase and specific activity of galactanase were 85.6% and 73.8%, respectively, compared to that of the unfiltered crude enzyme liquid derived from the filamentous fungus of the genus *Talaromyces* (Reference Example 2).

### <Example 6> Solid-liquid separation of a crude enzyme liquid derived from the filamentous fungus of the genus Talaromyces using a mixture of diatomaceous earth and a culture liquid containing cells of the filamentous fungus of the genus Talaromyces as a filter aid (precoat agent and body feed agent)

Diatomaceous earth "Celite 512" was added to the entire amount of the culture liquid of filamentous fungus of the genus *Talaromyces* prepared by the method described in Reference Example 1 to a concentration of 10% (w/v), and subjected to shaking culture at room temperature at 120 rpm for an hour to obtain a filter aid for precoating and body feeding. The crude enzyme liquid derived from the filamentous fungus of the genus *Talaromyces* was subjected to suction filtration to recover a filtrate in the same manner as in Comparative Example 4 except that this filter aid was used, and the protein concentration, specific activity of β-xylosidase, specific activity of pectinase, and specific activity of galactanase of the recovered filtrate were measured by the methods described in Reference Examples 4 to 7.

As shown in Table 4, specific activity of β-xylosidase decreased to 84.7% compared to that of the unfiltered crude enzyme liquid derived from the filamentous fungus of the genus *Talaromyces* (Reference Example 2). Further, specific activity of pectinase and specific activity of galactanase were 102% and 97.9%, respectively, compared to that of the unfiltered crude enzyme liquid derived from the filamentous fungus of the genus *Talaromyces* (Reference Example 2).

### [Table 4]

**[Table 4]**

| | Strain for preparing filter aid | Strain producing crude enzyme liquid | Composition of filter aid | Treatment | | Protein concentration | β-Xylosidase specific activity | Pectinase specific activity | Galactanase specific activity |
|---|---|---|---|---|---|---|---|---|---|
| | | | | Precoat | Body feed | Relative value (relative to Reference Example 2) | Relative value (relative to Reference Example 2) | Relative value (relative to Reference Example 2) | Relative value (relative to Reference Example 2) |
| Comparative Example 1 | - | *Trichoderma* | Celite 512 | Yes | No | 83.4 | 53.9 | - | - |
| Example 1 | *Trichoderma* | *Trichoderma* | Celite 512 + culture liquid | Yes | No | 91.0 | 74.7 | - | - |
| Example 2 | *Trichoderma* | *Trichoderma* | Celite 512 + supernatant of culture liquid | Yes | No | 83.8 | 68.1 | - | - |
| Comparative Example 2 | - | *Trichoderma* | Celite512 | Yes | Yes | 85.1 | 22.5 | - | - |
| Comparative Example 3 | *Trichoderma* | *Trichoderma* | Celite512 + fungal cells | Yes | Yes | 85.8 | 26.2 | - | - |
| Example 3 | *Trichoderma* | *Trichoderma* | Celite 512 + culture liquid | Yes | Yes | 79.6 | 88.4 | - | - |
| Example 4 | *Trichoderma* | *Trichoderma* | Celite 512 + supernatant of culture liquid | Yes | Yes | 86.7 | 67.8 | - | - |
| Example 5 | *Talaromyces* | *Trichoderma* | Celite 512 + culture liquid | Yes | Yes | 80.4 | 81.2 | - | - |
| Comparative Example 4 | - | *Talaromyces* | Celite 512 | Yes | Yes | 82.5 | 18.8 | 85.6 | 73.8 |
| Example 6 | *Talaromyces* | *Talaromyces* | Celite 512 + culture liquid | Yes | Yes | 104 | 84.7 | 102 | 97.9 |

### <Example 7> Saccharification test of cellulose-containing biomass

Saccharification tests of alkali-treated bagasse were performed using an unfiltered crude enzyme liquid derived from the filamentous fungus of the genus *Trichoderma* obtained in Reference Example 2, and filtrates obtained in Comparative Examples 2 and 3, and Examples 3 and 4.

An alkali-treated bagasse prepared according to the method described in Reference Example 4 as 5% (w/v) biomass was incubated at 50°C for 7 hours in a rotating rotor, and then a reaction supernatant was recovered by centrifugation at 20,000 ×g at 4°C for 10 minutes. To 180 µL of the reaction supernatant, 20 µL of 1 N NaOH was added as a reaction terminator liquid, and the amount of liberated xylose was quantified under the analytical conditions described in Reference Example 7.

As shown in Table 5, when the unfiltered crude enzyme liquid derived from the filamentous fungus of the genus *Trichoderma* (Reference Example 2) was used, the amount of xylose liberated was 10.1 g/L. When the filtrates of Comparative Examples 2 and 3 were used, the amounts of liberated xylose were 7.87 g/L and 8.04 g/L, respectively, which were reduced by 22.7% and 20.3%, respectively, compared to Reference Example 2. On the other hand, when the filtrate of Example 3 was used, the amount of liberated xylose was 11.5 g/L, which was increased by 13.9%, compared to Reference Example 2. When the filtrate of Example 4 was used, the amount of liberated xylose was 9.55 g/L, which was reduced by 5.44%, compared to from that in Reference Example 2, but the decrease remained less than 10%.

**[Table 5]**

| Crude enzyme liquid used | Amount of xylose liberated (g/L) | Change (%, relative to Reference Example 2) |
|---|---|---|
| Reference Example 2 | 10.1 | - |
| Comparative Example 2 | 7.87 | -22.7 |
| Comparative Example 3 | 8.04 | -20.3 |
| Example 3 | 11.5 | +13.9 |
| Example 4 | 9.55 | -5.44 |

## Claims

1. A filter aid which is a mixture of an inorganic filter aid and a culture liquid of a filamentous fungus.

2. The filter aid according to claim 1, wherein the inorganic filter aid is diatomaceous earth.

3. The filter aid according to claim 1 or 2, wherein the filamentous fungus is the genus *Trichoderma* or the genus *Talaromyces.*

4. The filter aid according to any one of claims 1 to 3, wherein the culture liquid is a culture liquid cultured until a dry cell weight (g-cell/L) of the filamentous fungal cells reaches at least 1.0 g/L or more.

5. The filter aid according to any one of claims 1 to 4, wherein the culture liquid contains filamentous fungal cells after culturing.

6. A method of filtration treatment, comprising a step of filtration treatment together with the filter aid according to any one of claims 1 to 5.

7. The method of filtration treatment according to claim 6, wherein the filtration treatment step is a step in which the filter aid according to any one of claims 1 to 5 is added to a liquid to be filtered.

8. The method of filtration treatment according to claim 6 or 7, wherein the filtration treatment step is a filtration treatment step in which the liquid to be filtered is passed through a filter medium precoated with the filter aid according to any one of claims 1 to 5 from a precoated side thereof.

9. A method of manufacturing cellulase, comprising:
a step (1) of culturing a microorganism capable of producing cellulase; and
a step (2) of performing filtration treatment of a culture liquid containing microorganism cells after culturing obtained in the step (1) together with the filter aid according to any one of claims 1 to 5, prepared independently of the step (1), to recover cellulase from which the microorganism cells have been filtered off.

10. The method of manufacturing cellulase according to claim 9, wherein the microorganism in the step (1) is a microorganism capable of producing cellulase having β-xylosidase activity, and the step (2) is a step of recovering cellulase having β-xylosidase activity.

11. The method of manufacturing cellulase according to claim 9 or 10, wherein the filtration treatment in the step (2) is a filtration treatment in which the filter aid according to any one of claims 1 to 5, prepared independently of the step (1), is added to the culture liquid containing microorganism cells after culturing obtained in the step (1).

12. The method of manufacturing cellulase according to any one of claims 9 to 11, wherein the filtration treatment in the step (2) is a filtration treatment in which the culture liquid containing microorganism cells after culturing obtained in the step (1) is passed through a filter medium precoated with the filter aid according to any one of claims 1 to 5, prepared independently of the step (1), from a precoated side thereof.

13. The method of manufacturing cellulase according to any one of claims 9 to 12, wherein the filtration treatment in the step (2) is a filter press treatment.

14. The method of manufacturing cellulase according to any one of claims 9 to 13, wherein the microorganism capable of producing cellulase, which is to be cultured in the step (1), is a filamentous fungus of the genus *Trichoderma* or the genus *Talaromyces.*

15. The method of manufacturing cellulase according to claim 14, comprising a step of mixing a preculture liquid of the filamentous fungus to be cultured in the step (1) and the inorganic filter aid to prepare the filter aid according to any one of claims 1 to 5.

16. A method of manufacturing a sugar, comprising:
a step of manufacturing cellulase by the method according to any one of claims 9 to 15; and
a step of hydrolyzing a cellulose-containing biomass with the cellulase obtained in the preceding step.
